# EUROPEAN PATENT APPLICATION

(11) **EP 4 607 193 A1**
(43) Date of publication of application: **27.08.2025**
(21) Application number: 23879881.3
(22) Date of filing: 19.10.2023
(51) Int. Cl.: G01N 33/53, G01N 33/543

(54) **WEARING ARTICLE PROVIDED WITH TEST STRIP FOR DETECTING AMNIOTIC FLUID MARKER**

(30) Priority: 21.10.2022 JP 2022169529
(71) Applicant: Cranebio Co., Ltd., Tokyo, 101-0021 (JP)
(72) Inventor: SAITO, Keita, Tokyo 101-0021 (JP); SUZUKI, Takeru, Tokyo 101-0021 (JP); TAKESHITA, Moto, Tokyo 101-0021 (JP); ABE, Misato, Tokyo 101-0021 (JP); SUGAWARA, Iku, Tokyo 101-0021 (JP)
(74) Representative: Mewburn Ellis LLP
(86) International application number: PCT/JP2023/037938
(87) International publication number: WO 2024/085238

(57) **Abstract**

Provided is a product that enables easy monitoring of early rupture of membranes and relates to a wearing article provided with a test strip using immunochromatography, wherein the test strip is designed to detect amniotic fluid-derived insulin-like growth factor binding protein 1 in vaginal secretion, and wherein, when the test strip detects 0.3-25 µg/L of insulin-like growth factor binding protein 1, the result is considered positive.

## Description

### FIELD

The present invention relates to a wearable article comprising an amniotic fluid marker detecting test strip and an amniotic fluid marker detecting test strip for use in a wearable article.

### BACKGROUND

Monitoring and early detection of membrane rupture, and in particular, premature rupture of membranes, is important for safe childbirth.

As a conventional testing method, for example, a pH testing cotton swab which is suitable for confirming amniotic fluid during premature rupture of membranes is known.

Kits based on antigen-antibody reaction for detecting IGFBP-1 derived from amniotic fluid are also known. In such kits, vaginal secretion are sampled using a sterile swab. In such conventional test kits, the detection range of IGFBP-1 is set to a relatively high concentration range. When such a test kit is provided in the form of a liner, IGFBP-1 is diluted by compounds contained in other mucus, but since the dilution rate is unknown, it is necessary to readjust the sensitivity.

Panty liners for detecting amniotic fluid leakage which function by detecting changes in pH, which are believed to be indicative of amniotic fluid leakage, are also known.

### SUMMARY

### [TECHNICAL PROBLEM]

In the conventional methods, in order to confirm the presence or absence of premature rupture of membranes, a cotton swab, etc. is used to swab the vagina and sample the vaginal secretion serving as the detection target. This detection method is time-consuming and not suitable for applications in which continuous monitoring is performed.

Furthermore, since it is difficult for premature rupture of membranes to be recognized by the individual themself, there may be no opportunity to start monitoring.

An object of the present invention is to provide a product with which monitoring of premature rupture of membranes can conveniently be performed.

### [SOLUTION TO PROBLEM]

The object of the present invention can be achieved by the present invention, which includes the following aspects:

### <Aspect 1>

A wearable article, comprising a test strip using immunochromatography, wherein
the test strip is for detecting amniotic fluid-derived insulin-like growth factor binding protein 1 in vaginal secretion, and
the test strip detects 0.3 µg/L to 25 µg/L of insulin-like growth factor binding protein 1 as positive.

### <Aspect 2>

The wearable article according to Aspect 1, which is an undergarment or absorbent article.

### <Aspect 3>

The wearable article according to Aspect 1 or 2, wherein the test strip has a width of 3 mm or less.

### <Aspect 4>

The wearable article according to any one of Aspects 1 to 3, wherein the test strip comprises:
a first member which contacts vaginal secretion,
a second member which contains a labeled antibody that recognizes amniotic fluid-derived insulin-like growth factor binding protein 1 in vaginal secretion, and
a third member which has a display part which contains a capture antibody,
the capture antibody is capable of capturing a complex formed by binding of the labeled antibody and insulin-like growth factor binding protein 1, and
the test strip is configured such that the amniotic fluid-derived insulin-like growth factor binding protein 1 in vaginal secretion moves from the first member, through the second member, to the display part of the third member, and that
the display part of the third member changes color when the capture antibody captures the complex.

### <Aspect 5>

The wearable article according to Aspect 4, wherein the first member, the second member, and the third member are stacked in this order at least partially overlapping each other, and
a sum of thicknesses of the first member, the second member, and the third member in the stacking direction is 1.5 mm or less.

### <Aspect 6>

The wearable article according to Aspect 4 or 5, wherein the labeled antibody comprises a colorant having a particle diameter of 250 nm to 500 nm.

### <Aspect 7>

A test strip using immunochromatography for use in a wearable article, wherein
the test strip is for detecting amniotic fluid-derived insulin-like growth factor binding protein 1 in vaginal secretion, and
the test strip detects 0.3 µg/L to 25 µg/L of insulin-like growth factor binding protein 1 as positive.

### [ADVANTAGEOUS EFFECTS OF INVENTION]

According to the present invention, there can be provided a product with which monitoring of premature rupture of membranes can conveniently be performed.

### BRIEF DESCRIPTION OF DRAWINGS

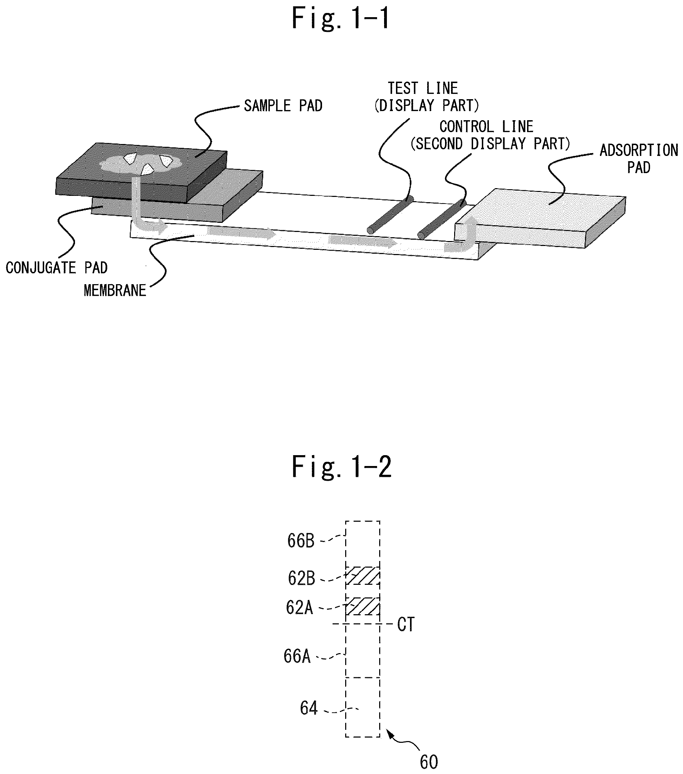

FIG. 1-1 is a conceptual diagram showing a specific configuration of a test strip according to the present invention.
FIG. 1-2 is a schematic view showing a specific configuration of a test strip according to the present invention.
FIG. 2-1 is a view detailing a reference example.
FIG. 2-2 is a view detailing a reference example.

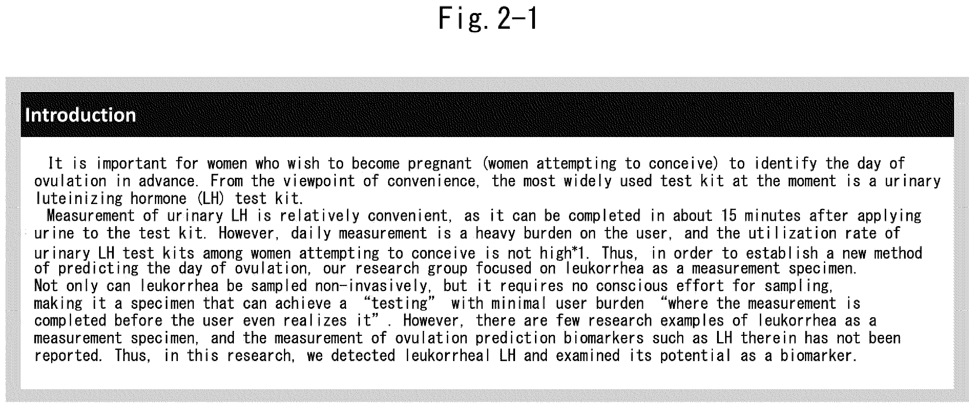

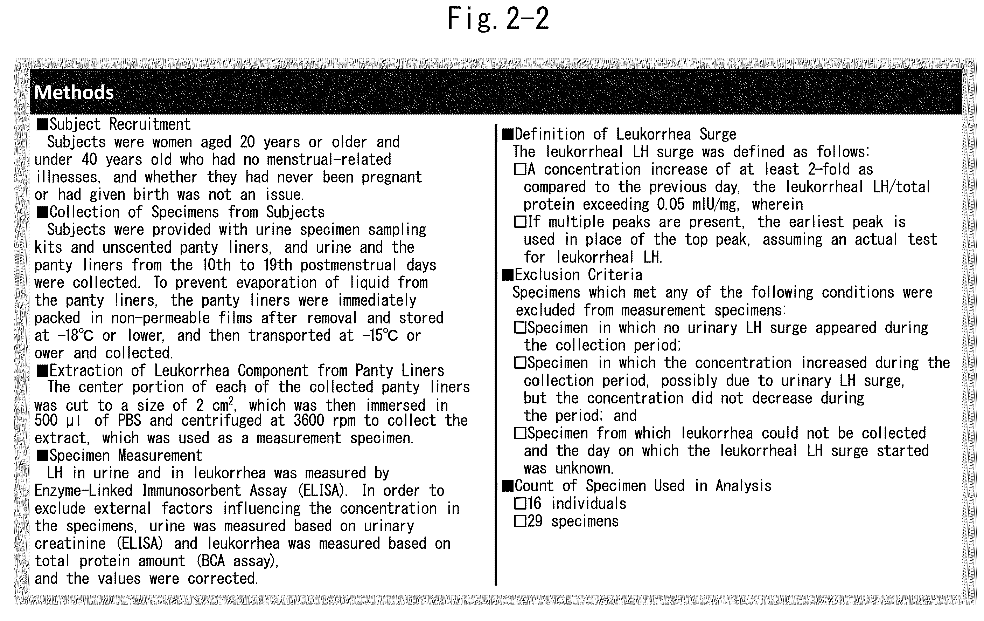

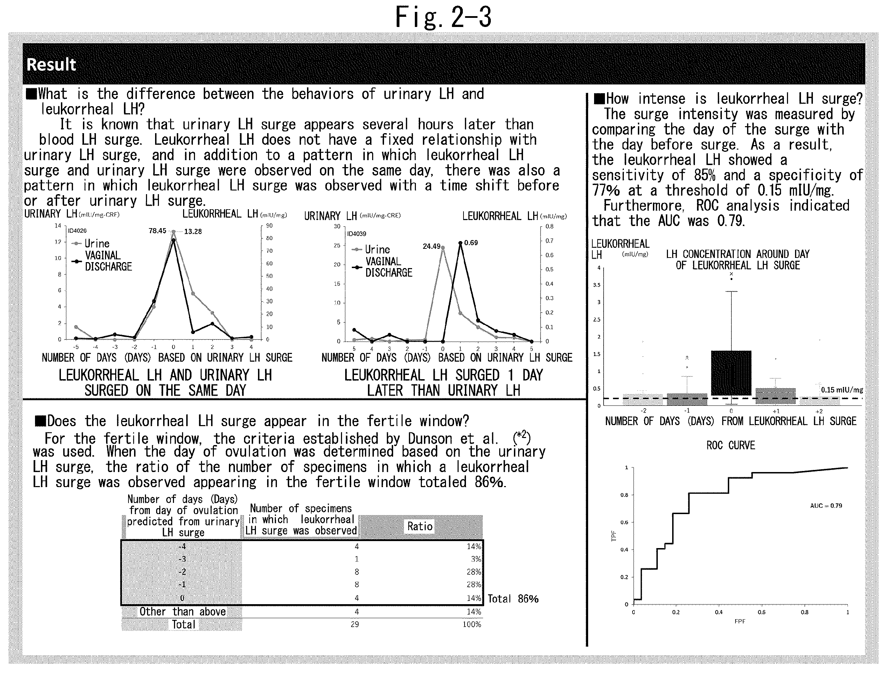

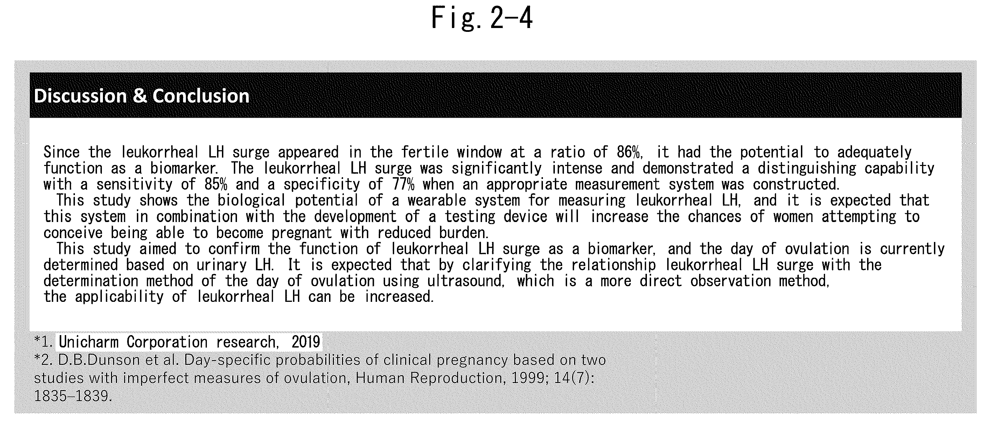

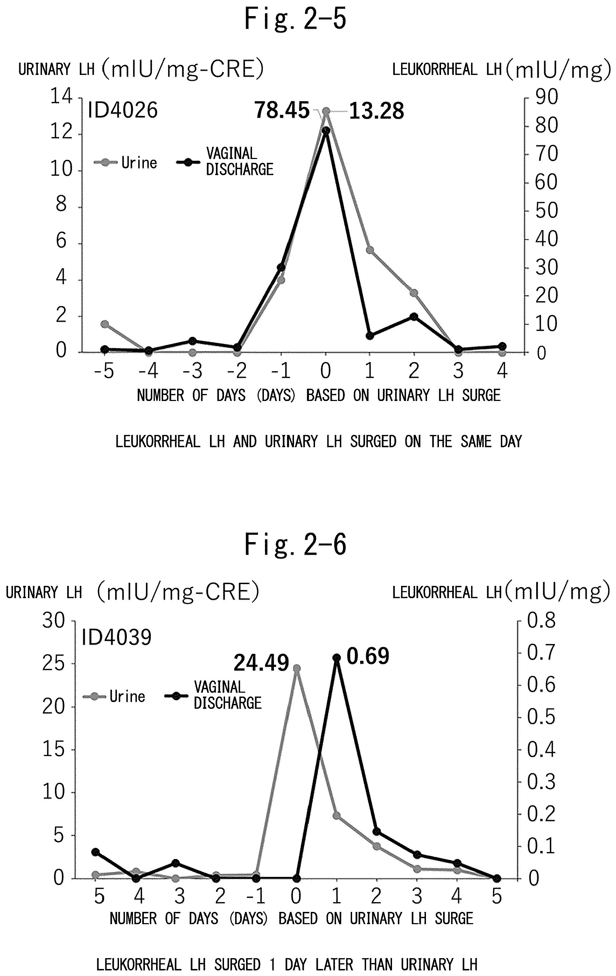

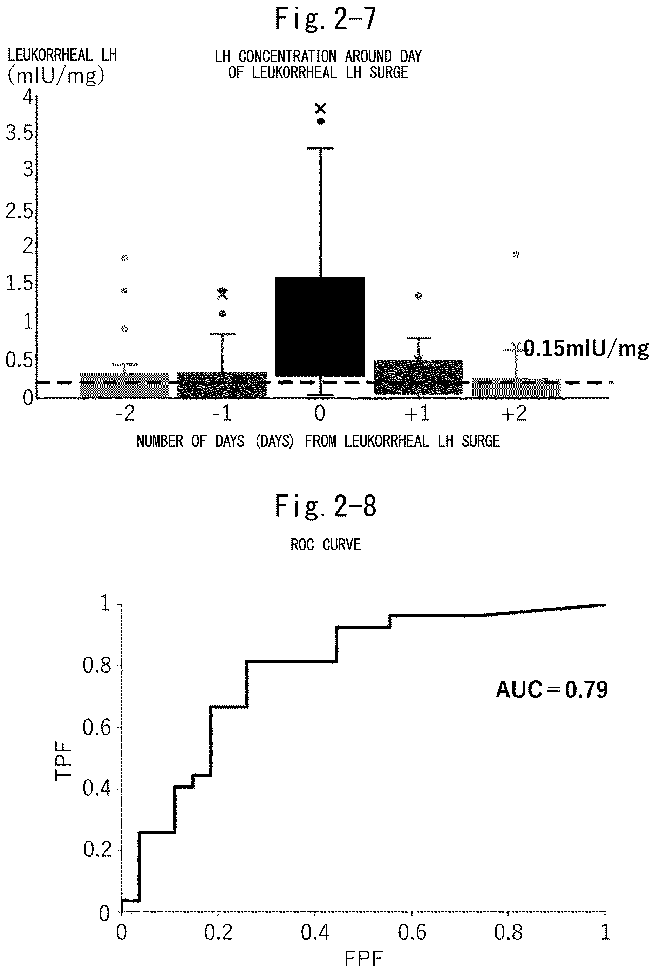

FIG. 2-3 is a view detailing a reference example.
FIG. 2-4 is a view detailing a reference example.
FIG. 2-5 is an expanded view of one of the graphs shown in FIG. 2-3.
FIG. 2-6 is an expanded view of one of the graphs shown in FIG. 2-3.
FIG. 2-7 is an expanded view of one of the graphs shown in FIG. 2-3.
FIG. 2-8 is an expanded view of one of the graphs shown in FIG. 2-3.

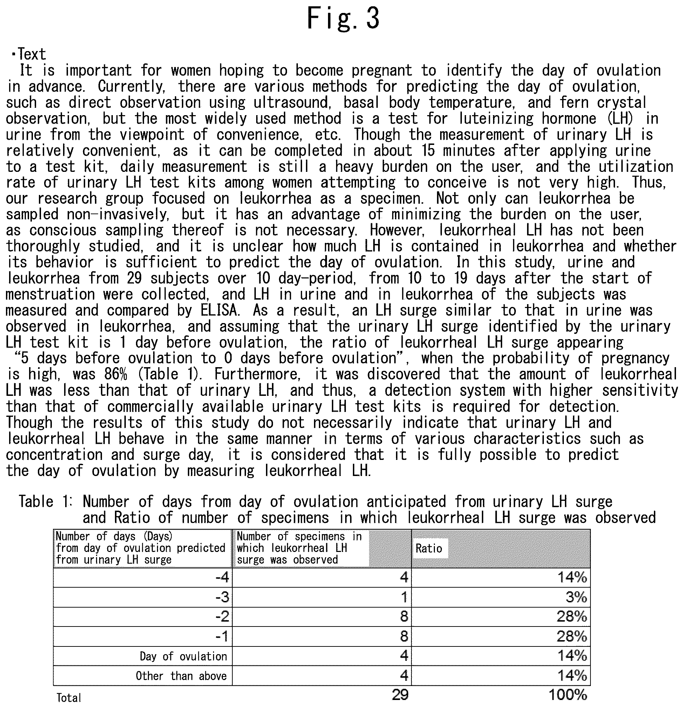

FIG. 3 is a view detailing a reference example.

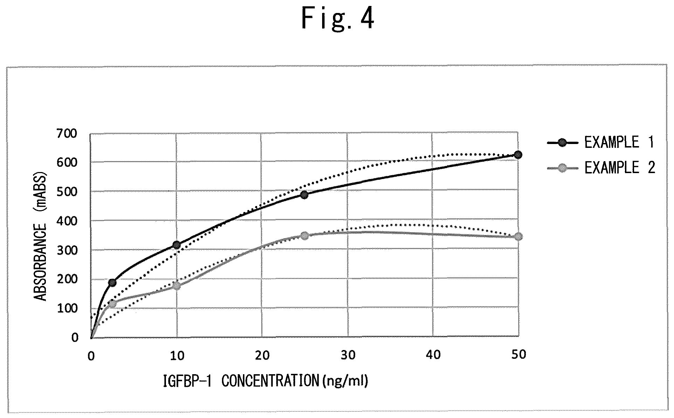

FIG. 4 is a graph showing the results of a sensitivity test of the test strips according to Examples 1 and 2.

### DESCRIPTION OF EMBODIMENTS

The present invention will be described in detail below.

### <Aspect 1>

A wearable article, comprising a test strip using immunochromatography, wherein
the test strip is for detecting amniotic fluid-derived insulin-like growth factor binding protein 1 in vaginal secretion, and
the test strip detects 0.3 µg/L to 25 µg/L of insulin-like growth factor binding protein 1 as positive.

As described above, in conventional methods, in order to confirm the presence or absence of premature rupture of membranes, it is necessary to sample vaginal secretion by swabbing the vagina with a cotton swab, etc. However, since it is difficult for premature rupture of membranes to be recognized by the individual themself, in such time-consuming methods, there is a risk that premature rupture of membranes may not be detected early.

In contrast, in the invention according to Aspect 1 of the present application, since a test strip for detecting insulin-like growth factor binding protein 1 (IGFBP-1), which is contained in large amounts in amniotic fluid, is applied to the wearable article, monitoring of premature rupture of membranes can be performed simply by wearing the article without the need for any special operations.

Furthermore, in the test strip included in the wearable article according to Aspect 1 of the present application, since the detection concentration range is optimized to 0.3 µg/L to 25 µg/L, amniotic fluid-derived insulin-like growth factor binding protein 1 in vaginal secretion can be detected with high reliability.

The detection concentration of the test strip can be measured using a casein blocking buffer solution of IGFBP1 (1.0% casein, 100 mM borate, pH 8.5) as a standard solution.

Though not intended to be limiting, for example, a standard solution can be prepared using insulin-like growth factor binding protein 1 (IGFBP1), human, recombinant (25 µg) (Fujifilm Wako Pure Chemical Corporation: product code 092-06201) as a positive specimen powder and a blocking buffer solution (1.0% casein, 100 mM borate, pH 8.5) as the dilution solution, and dissolving and diluting the positive specimen powder with the dilution solution to the object concentration.

The test strip of the present invention detects:
more preferably, 0.4 µg/L to 20 µg/L;
further preferably, 0.5 µg/L to 15 µg/L; and
even further preferably, 0.5 µg/L to 10 µg/L of insulin-like growth factor binding protein 1 as positive.

### <Aspect 2>

The wearable article according to Aspect 1, which is an undergarment or absorbent article.

In the invention according to Aspect 2, since the wearable article is an undergarment or absorbent article that can be worn on a daily basis, monitoring of premature rupture of membranes can be performed relatively conveniently and continuously.

In this aspect, for example, the test strip may be bonded to the inner surface of the undergarment, or the test strip may be arranged between the sheet-like members constituting the absorbent article. The method for applying the test strip to a wearable article such as an absorbent article is not particularly limited, and can be performed according to a known method.

### <Aspect 3>

The wearable article according to Aspect 1 or 2, wherein the test strip has a width of 3 mm or less.

In the cases of premature rupture of membranes, the amount of fluid may be relatively small. Furthermore, in cases of premature rupture of membranes, amniotic fluid may be discharged together with vaginal discharge, etc., and in such cases, the viscosity of the amniotic fluid-containing vaginal secretion which come into contact with the test strip may be relatively high.

In contrast, in the invention according to Aspect 3, the width of the test strip is reduced to 3 mm or less, and the amount of fluid necessary to be retained in the test strip for detection is relatively reduced. Thus, in the invention according to Aspect 3, even when amniotic fluid-containing vaginal secretion, which are low in volume and high in viscosity, are to be detected, detection can be satisfactorily performed.

Note that the width of the test strip may be 1 mm to 10 mm, but is preferably 5 mm or less. The upper limit of the width of the test strip may further be 4 mm or less. The width of the test strip is particularly preferably 1 mm or more and less than 5 mm, 1.5 mm to 4.5 mm, or further preferably 2 mm to 4 mm, and particularly preferably 2 mm to 3 mm. When the width of the test strip is within these ranges, particularly suitable visibility of the color reaction, etc., may be obtained.

From the viewpoints of visibility for the user and ease of processing, the width of the test strip is preferably 2 mm or more.

### <Aspect 4>

The wearable article according to any one of Aspects 1 to 3, wherein the test strip comprises:
a first member which contacts vaginal secretion,
a second member which contains a labeled antibody that recognizes amniotic fluid-derived insulin-like growth factor binding protein 1 in vaginal secretion, and
a third member which has a display part which contains a capture antibody,
the capture antibody is capable of capturing a complex formed by binding of the labeled antibody and insulin-like growth factor binding protein 1, and
the test strip is configured such that the amniotic fluid-derived insulin-like growth factor binding protein 1 in vaginal secretion moves from the first member, through the second member, to the display part of the third member, and that
the display part of the third member changes color when the capture antibody captures the complex.

In the wearable article according to Aspect 4, detection of insulin-like growth factor binding protein 1 can be performed conveniently and quickly via a color reaction.

When the wearable article is an undergarment, for example, the color change can be confirmed by direct visual confirmation of the test strip bonded to the inner surface of the undergarment. Furthermore, when the wearable article is an absorbent article (for example, a panty liner), for example, color change in the test strip, which is arranged between the sheet-like members constituting the absorbent article, can be confirmed via a visible light-transmitting sheet-like member.

The measurement principle of the test strip according to Aspect 4 will be described using a specific example. In an exemplary aspect, for example, the labeled antibody is a colorant-labeled mouse anti-human insulin-like growth factor binding protein type 1 monoclonal antibody, and the capture antibody is a mouse anti-human insulin-like growth factor binding protein type 1 monoclonal antibody. If IGFBP-1 is present in a specimen which has contacted the first member, the labeled antibody reacts with the IGFBP-1 in the second member to form a complex based on the antigen-antibody reaction. This complex moves to the display part of the third member and is captured by the capture antibody immobilized and bound there, and as a result, the display part changes color based on the colorant.

A second display part (control part) can be provided downstream of the display part in the direction of antigen movement. A second capture antibody (for example, rabbit anti-mouse immunoglobulin polyclonal antibody) different from the capture antibody described above is immobilized and bound to this control part. This second capture antibody can recognize the labeled antibody. Labeled antibodies which do not form a complex with the antigen pass through the display part without being captured by the display part, and are captured by the control part, resulting in the control part changing color based on a colorant.

According to this aspect, in the case of a positive result, the display part and the control part change color, and in the case of a negative result, only the control part changes color.

The configuration of the test strip will be described in detail later.

### <Aspect 5>

The wearable article according to Aspect 4, wherein the first member, the second member, and the third member are stacked in this order at least partially overlapping each other, and
a sum of thicknesses of the first member, the second member, and the third member in the stacking direction is 1.5 mm or less.

As described above, in cases of premature rupture of membranes, amniotic fluid may be discharged together with vaginal discharge, etc., and in such cases, the viscosity of the amniotic fluid-containing vaginal secretion which come into contact with the test strip may be relatively high. Thus, it was sometimes difficult to detect amniotic fluid-derived insulin-like growth factor binding protein 1 in vaginal secretion using conventional detection methods using immunochromatography.

In contrast, in the test strip according to Aspect 5, since the thicknesses of the members constituting the test strip are reduced, the amount of liquid necessary to be retained in the test strip for detection is relatively reduced. Thus, in the test strip according to Aspect 5, even when amniotic fluid-containing vaginal secretion, which are low in volume and high in viscosity, are detected, detection of insulin-like growth factor binding protein 1 can be satisfactorily performed.

The preferred range of membrane thickness is 160 to 230 µm, and more preferably 180 to 220 µm.

### <Aspect 6>

The wearable article according to Aspect 4 or 5, wherein the labeled antibody comprises a colorant having a particle diameter of 250 nm to 500 nm (preferably 280 to 450 nm, and further preferably 300 nm to 400 nm).

As described above, in cases of premature rupture of membranes, amniotic fluid may be discharged together with vaginal discharge, etc., and in this case, the viscosity of the amniotic fluid-containing vaginal secretion which come into contact with the test strip may be relatively high. When the viscosity of the amniotic fluid-containing vaginal secretion is relatively high, the fluidity of the specimen in the test strip may decrease, and the absolute amount of antigen used for detection may be relatively small. Furthermore, the amount of amniotic fluid contained in the vaginal secretion may be small.

In contrast, in the wearable article according to Aspect 6, since the labeled antibody which binds to the insulin-like growth factor binding protein 1 comprises a colorant having a relatively large particle diameter, detection visibility is improved. Thus, in the wearable article according to Aspect 6, detection can be suitably performed even when the amount of antigen is relatively small due to the viscosity and/or amount of amniotic fluid-containing vaginal secretion.

### <Aspect 7>

A test strip using immunochromatography for use in a wearable article, wherein
the test strip is for detecting amniotic fluid-derived insulin-like growth factor binding protein 1 in vaginal secretion, and
the test strip detects 0.3 µg/L to 25 µg/L of insulin-like growth factor binding protein 1 as positive.

As described above, in conventional methods, in order to confirm the presence or absence of premature rupture of membranes, it is necessary to sample vaginal secretion by swabbing the vagina with a cotton swab, etc. However, since it is difficult for premature rupture of membranes to be recognized by the individual themself, in such time-consuming methods, there is a risk that premature rupture of membranes may not be detected early.

In contrast, in the invention according to Aspect 7, since a test strip for detecting insulin-like growth factor binding protein 1 (IGFBP-1), which is found in large amounts in amniotic fluid, is applied to the wearable article, monitoring of membrane rupture (and in particular, premature rupture of membranes) can be performed simply by wearing the article without the need for any special operations.

Furthermore, according to this test strip, since the detection concentration range is optimized to 0.3 µg/L to 25 µg/L, amniotic fluid-derived insulin-like growth factor binding protein 1 in vaginal secretion can be detected with high reliability.

Note that regarding the details of the test strip according to Aspect 7, reference can be made to the descriptions of the wearable articles according to Aspects 1 to 6 (and in particular, the descriptions regarding the test strip).

### <Specific Examples of Test Strip>

A conceptual diagram of a test strip is shown in the attached FIG. 1-1. Regarding the other configurations of the test strip, reference can be made to, for example, WO2021/132724A1. Regarding an absorbent article comprising a test strip, reference can be made to, for example, WO2021/132724.

A test strip using immunochromatography will be specifically described with reference to the drawings. Note that the drawings are not necessarily to scale and are not intended to limit the present invention.

The test strip (test member) 60 of FIG. 1-2 has a display part (62A, 62B) which can change color based on excretions (vaginal secretion). The test strip 60 has a contact part (first member, for example, a sample pad) 64 which contacts the vaginal secretion. The test strip 60 may have a moving part through which components contained in the vaginal secretion (hereinafter also referred to as "vaginal secretion components") move. In the moving part, the vaginal secretion components may move by capillary action. The moving part may have a display moving part 66A through which the vaginal secretion components move from the contact part 64 to the display part (62A, 62B), and a terminal moving part 66B through which the vaginal secretion components move in a direction away from the display part (62A, 62B). The terminal moving part is a part through which the vaginal secretion components and others that have passed through the display part (62A, 62B) move.

The contact part (first member) 64 with which the vaginal secretion come into contact may be composed of, for example, a pad (which may be referred to as a sample pad or specimen pad). The vaginal secretion components in the vaginal secretion which have come into contact with the contact part 64 move, for example, through the pad and migrate to a second member (for example, a conjugate pad). The conjugate pad contains a labeled antibody which recognizes amniotic fluid-derived insulin-like growth factor binding protein 1 in vaginal secretion. Though the conjugate pad (second member) is not illustrated in FIG. 3-1, it may be arranged so as to overlap the sample pad (first member) in the thickness direction. The vaginal secretion components, the complex formed by the antigen-antibody reaction with the amniotic fluid-derived insulin-like growth factor binding protein 1 contained in the vaginal secretion components, and the labeled antibody may move through the moving part 66A by capillary action. The part where these substances move may be composed of, for example, a membrane. The display part has an area 62A containing a capture antibody for capturing (binding) the formed complex. This area changes color when the complex is captured by the capture antibody. This area 62A of the display part may be referred to as a test line.

The display part may have an area 62B containing a capture antibody in addition to the area 62A containing the capture antibody for capturing the complex. The capture antibody in the area 62B may capture a complex other than the complex captured in the area 62A, or may capture a labeled antibody. The area 62B changes color when a complex or the labeled antibody is captured. The area 62B for capturing the labeled antibody may be referred to as a control line. The vaginal secretion components which have passed through the display part 62 (i.e., the components which have not been captured by the capture antibody) move through the terminal moving part 66B. The terminal moving part 66B may be composed of a membrane. The terminal moving part 66B may also have an adsorption pad for absorbing the vaginal secretion components that have passed through the display part 62. The vaginal secretion components which have passed through the display part 62 may be absorbed by the absorbent core instead of the adsorption pad. When the immunochromatography is used, the display part 62 and the contact part 64 are arranged at different positions. Thus, in this case, the display part 62 and the contact part 64 are different.

The test strip (test member) 60 needs only to comprise members which are capable of retaining or containing substances such as labeled antibodies and capture antibodies, and may be composed of any of the materials such as paper, nonwoven fabric, or woven fabric.

### <Method for Detecting Amniotic Fluid-Derived Insulin-Like Growth Factor Binding Protein 1 From Vaginal Secretion>

The present disclosure includes a method for detecting amniotic fluid-derived insulin-like growth factor binding protein 1 in vaginal secretion using a wearable article comprising a test strip using immunochromatography, the method comprising:
allowing vaginal secretion to adhere to the test strip, wherein
in the test strip, 0.3 µg/L to 25 µg/L of insulin-like growth factor binding protein 1 is detected as positive.

Regarding details of each constituent element in this method, such as the wearable article and the test strip, reference can be made to the above descriptions of the wearable article and the test strip.

The method of allowing the vaginal secretion to adhere to the test strip is not particularly limited, and reference can be made to the above descriptions of the wearable article and the test strip. For example, the wearable article can be configured so that the vaginal secretion excreted from the wearer wearing the wearable article adhere to the test strip (in particular, the first member of the test strip). Furthermore, for example, in the test strip of Aspect 4 above, vaginal secretion can adhere to the test strip by configuring the wearable article so that vaginal secretion directly adhere to the first member (in particular, the sample pad). Preferably, the test strip is configured so as to be in direct contact with the vagina (vulva).

In an embodiment of the method described above, 0.4 µg/L to 20 µg/L, or 0.5 µg/L to 15 µg/L, or 0.5 µg/L to 10 µg/L of insulin-like growth factor binding protein 1 is detected as positive.

### <Reference Examples>

For reference, attached FIGS. 2-1 to 2-8 and FIG. 3 show experimental results regarding the detection of a substance (LH) in vaginal discharge.

### <<Examples 1 and 2>>

In Examples 1 and 2, a test strip for detecting amniotic fluid-derived insulin-like growth factor binding protein 1 in vaginal secretion using immunochromatography was prepared, and a sensitivity test was performed. Furthermore, the test strip was applied to a wearable article, and a wearing test was performed.

### (Example 1)

The test strip of Example 1 comprised:
a first member which contacted vaginal secretion,
a second member which contained a labeled antibody (manufactured by Medix Biochemica) that recognized amniotic fluid-derived insulin-like growth factor binding protein 1 (IGFBP-1) in vaginal secretion, and
a third member which had a display part which contained a capture antibody,
the capture antibody of the test strip in Example 1:
   was capable of capturing a complex formed by binding of the labeled antibody and insulin-like growth factor binding protein 1, and
   the test strip was configured such that the amniotic fluid-derived insulin-like growth factor binding protein 1 in vaginal secretion moved from the first member, through the second member, to the display part of the third member, and that
   the display part of the third member changed color when the capture antibody captured the complex.

The length of the test strip in Example 1 was 12 mm, and the width thereof was 3 mm. The thickness of the sample pad as the first member was 0.1 mm, the thickness of the conjugate pad as the second member was 0.55 mm, the thickness of the membrane as the third member was 0.1 mm, and the total thickness of the first to third members was 0.75 mm.

The labeled antibody in the conjugate pad of Example 1 contained blue nanobeads having a particle diameter of 350 nm (product name: NanoAct, manufactured by Asahi Kasei Corporation) as a colorant.

### (Example 2)

The overall configuration and dimensions of the test strip of Example 2 and the dimensions of the first to third members were the same as those of Example 1.

The labeled antibody in the conjugate pad of Example 2 contained blue nanobeads having a particle diameter of 350 nm (product name: NanoAct, manufactured by Asahi Kasei Corporation) as a colorant.

### <Sensitivity Test>

A sensitivity test was performed on the test strips of Examples 1 and 2. Specifically, IGFBP-1 solutions having concentrations of 2.5 ng/ml, 10 ng/ml, 25 ng/ml, and 50 ng/ml were prepared, and the sensitivities thereof after each was applied to the test strips was measured using an absorbance meter (product name: Immunochromato Reader (C10066-10), manufactured by Hamamatsu Photonics K.K.). The results are shown in the graph of FIG. 4.

As can be seen from the graph of FIG. 4, the test strips of Examples 1 and 2 could detect IGFBP-1 at relatively low concentrations (for example, 0.5 to 10 µg/L) with very high sensitivity. These test strips could also reliably detect trace amounts of amniotic fluid presence.

Furthermore, tests in which pregnant women actually wore articles to which the test strips according to Examples 1 and 2 had been adhered were also performed. As a result, no false positives were observed, and suitable detection was achieved.

### <<Examples 3 and 4>>

In Examples 3 and 4, the relationship between the width of the test strip and the visibility of the color change was examined. The test strip of Example 3 had a width of 5 mm, and the test strip of Example 4 had a width of 3 mm. The width of the test strip means the length in the overall plane of the test strip in the direction perpendicular to the length of the test strip (in particular, the direction in which the vaginal secretion components travel).

Vaginal secretion sampled from each of three subjects over 10 to 12 days were allowed to adhere to the sample pad of the test strip of Example 3 or 4, which had a similar configuration as in Example 1 described above, and the color change at the display part was observed. The total number of tests performed was n=25 for Example 3 and n=28 for Example 4.

Regarding the color change at the display part, the ratio of cases in which the control line was clearly visible (Excellent) and cases in which the control line could be visually confirmed but with slightly poor visibility (Poor) was calculated. The results are shown in Table 1 below.

### [Table 1]

**Table 1**

| | Test strip width | Total sample number (n) | Visibility evaluation | | | |
|---|---|---|---|---|---|---|
| | | | Excellent | Poor | Ratio of excellent | Ratio of poor |
| Example 3 | 5 mm | 25 | 16 | 9 | 64% | 36% |
| Example 4 | 3 mm | 28 | 24 | 4 | 86% | 14% |

As can be seen from Table 1, the test strip of Example 4, which had a width of 3 mm, exhibited superior visibility to the test strip of Example 3, which had a width of 5 mm.

Without intending to be limited by theory, it is believed that the flow of the sample was improved by the relatively narrow width of the test strip. Specifically, it is believed that the test strip of Example 4 had improved visibility as a result of a relatively increased amount of vaginal secretion (and their components) components reaching the display part, which was the color-changing area.

## Claims

1. A wearable article, comprising a test strip using immunochromatography, wherein
the test strip is for detecting amniotic fluid-derived insulin-like growth factor binding protein 1 in vaginal secretion, and
the test strip detects 0.3 µg/L to 25 µg/L of insulin-like growth factor binding protein 1 as positive.

2. The wearable article according to claim 1, which is an undergarment or absorbent article.

3. The wearable article according to claim 1 or 2, wherein the test strip has a width of 3 mm or less.

4. The wearable article according to claim 1 or 2, wherein the test strip comprises:
a first member which contacts vaginal secretion,
a second member which contains a labeled antibody that recognizes amniotic fluid-derived insulin-like growth factor binding protein 1 in vaginal secretion, and
a third member which has a display part which contains a capture antibody,
the capture antibody is capable of capturing a complex formed by binding of the labeled antibody and insulin-like growth factor binding protein 1, and
the test strip is configured such that the amniotic fluid-derived insulin-like growth factor binding protein 1 in vaginal secretion moves from the first member, through the second member, to the display part of the third member, and that
the display part of the third member changes color when the capture antibody captures the complex.

5. The wearable article according to claim 4, wherein the first member, the second member, and the third member are stacked in this order at least partially overlapping each other, and
a sum of thicknesses of the first member, the second member, and the third member in the stacking direction is 1.5 mm or less.

6. The wearable article according to claim 4, wherein the labeled antibody comprises a colorant having a particle diameter of 250 nm to 500 nm.

7. A test strip using immunochromatography for use in a wearable article, wherein
the test strip is for detecting amniotic fluid-derived insulin-like growth factor binding protein 1 in vaginal secretion, and
the test strip detects 0.3 µg/L to 25 µg/L of insulin-like growth factor binding protein 1 as positive.
